# EUROPEAN PATENT SPECIFICATION

(11) **EP 4 582 029 B1**
(45) Date of publication and mention of the grant of the patent: **09.12.2026**
(21) Application number: 25172315.1
(22) Date of filing: 05.10.2022
(51) Int. Cl.: A61B 8/12, A61B 8/00, A61B 8/08

(54) **MEDICAL DEVICE SYSTEMS FOR AUTOMATED LESION ASSESSMENT**
MEDIZINISCHE VORRICHTUNGSSYSTEME ZUR AUTOMATISCHEN LÄSIONSBEWERTUNG
SYSTÈMES DE DISPOSITIF MÉDICAL POUR ÉVALUATION AUTOMATIQUE DE LÉSION

(30) Priority: 08.10.2021 US 202163253881 P
(43) Date of publication of application: 09.07.2025
(62) Document number(s) of the earlier application(s) in accordance with Art. 76 EPC: 22809937.0 / 4 398 805
(73) Proprietor: Boston Scientific Scimed, Inc., Maple Grove, MN 55304 (US)
(72) Inventor: FREED, Erik, New Brighton, Minnesota 55112 (US); CAI, Anming, San Jose, California 95120 (US); HUSTON, Matthew, Maple Grove, Minnesota 55369 (US); GIBBS, Jennifer, White Bear, Minnesota 55110 (US); MORLOCK, Jessica, Hopkins, Minnesota 55343 (US); DRURY, Heather, Springs, Texas 78620 (US); LI, Wenguang, Los Gatos, California 95032 (US)
(74) Representative: Vossius & Partner Patentanwälte Rechtsanwälte mbB

(56) References cited:
- WO-A1-2021/062006
- US-A1- 2019 282 182
- US-A1- 2020 029 932

## Description

### Technical Field

The present disclosure pertains to medical devices and/or medical device systems. More particularly, the present disclosure pertains to medical device systems for automatic lesion assessment.

### Background

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

WO 2021/062006 A1 relatesd to methods including methods for processing intravascular ultrasound images.

US 2019/282182 A1 relates to systems, devices, and methods to determine and visualize landmarks with an intraluminal imaging system for lesion assessment and treatment. An intraluminal imaging system may be configured to receive imaging data from an intraluminal imaging device, generate a set of image frames using received imaging data, and automatically calculate a luminal area associated with the body lumen for each of the image frames. The calculated luminal area and a longitudinal view of the body lumen may be displayed to a user on a display device.

US 2020/029932 A1 relates to systems, devices, and methods for displaying multiple intraluminal images in a luminal assessment. An intraluminal imaging device is provided which is configured to be positioned within a body lumen of a patient and receive imaging data associated with the body lumen. The intraluminal imaging device may be in communication with a controller configured to display two or more transverse images of the body lumen on a single display device. The system may also be configured to automatically measure and display measurements of anatomical features.

The invention is set out in the appended set of claims.

### Brief Summary of the Disclosure

This disclosure provides design, material, manufacturing method, and use alternatives for medical devices. An example intravascular imaging system is disclosed. The intravascular imaging system comprises: a catheter including an imaging device; a processor coupled to the catheter, the processor configured to process imaging data received from the imaging device; wherein the processor is configured to generate a longitudinal section view of a blood vessel from the imaging data received from the imaging device; wherein the processor is configured to identify a minimum lumen area along the longitudinal section view of the blood vessel, a distal reference point, and a proximal reference; and a display unit coupled to the processor, the display unit being configured to show a display including the longitudinal section view of the blood vessel.

Alternatively or additionally to any of the embodiments above, the display includes an indicator of the minimum lumen area.

Alternatively or additionally to any of the embodiments above, the indicator of the minimum lumen area is movable along the longitudinal section view of the blood vessel by a user.

Alternatively or additionally to any of the embodiments above, the display includes an indicator of the distal reference point.

Alternatively or additionally to any of the embodiments above, the indicator of the distal reference point is movable along the longitudinal section view of the blood vessel by a user.

Alternatively or additionally to any of the embodiments above, the display includes an indicator of the proximal reference point.

Alternatively or additionally to any of the embodiments above, the indicator of the proximal reference point is movable along the longitudinal section view of the blood vessel by a user.

Alternatively or additionally to any of the embodiments above, the processor is configured to identify a second minimum lumen area along the longitudinal section view of the blood vessel.

Alternatively or additionally to any of the embodiments above, the processor is configured to identify a secondary reference point along the longitudinal section view of the blood vessel.

An intravascular imaging system is disclosed. The intravascular imaging system comprises: an imaging catheter; a processor coupled to the catheter, the processor configured to process imaging data received from the imaging catheter; wherein the processor is configured to generate a longitudinal section view of a blood vessel from the imaging data; wherein the processor is configured to identify a minimum lumen area position along the longitudinal section view of the blood vessel, a distal reference point disposed distal of the minimum lumen area position, and a proximal reference disposed proximal of the minimum lumen area position; a display unit coupled to the processor, the display unit being configured to show a display; wherein the display includes the longitudinal section view of the blood vessel; and wherein the display includes an indicator of the minimum lumen area position, an indicator of the distal reference point, and an indicator of the proximal reference point along the longitudinal section view of the blood vessel.

Alternatively or additionally to any of the embodiments above, the indicator of the minimum lumen area position is movable along the longitudinal section view of the blood vessel by a user.

Alternatively or additionally to any of the embodiments above, the indicator of the distal reference point is movable along the longitudinal section view of the blood vessel by a user.

Alternatively or additionally to any of the embodiments above, the indicator of the proximal reference point is movable along the longitudinal section view of the blood vessel by a user.

Alternatively or additionally to any of the embodiments above, the processor is configured to identify a second minimum lumen area along the longitudinal section view of the blood vessel.

Alternatively or additionally to any of the embodiments above, the processor is configured to identify a secondary reference point along the longitudinal section view of the blood vessel.

A method for processing imaging data is disclosed. The method comprises: obtaining imaging data from an imaging catheter; processing the imaging data with a processor to generate a longitudinal section view of a blood vessel; wherein processing the imaging data includes identifying a minimum lumen area position of the blood vessel along the longitudinal section view of the blood vessel, identifying a distal reference point of the blood vessel along the longitudinal section view of the blood vessel, and identifying a proximal reference point of the blood vessel along the longitudinal section view of the blood vessel; modifying one or more of the minimum lumen area position, the distal reference point, and the proximal reference point to a secondary position; and affirming the secondary position.

Alternatively or additionally to any of the embodiments above, modifying one or more of the minimum lumen area position, the distal reference point, and the proximal reference point to a secondary position includes translating a bookmark corresponding to the minimum lumen area position, the distal reference point, or the proximal reference point along the longitudinal section view of the blood vessel to the secondary position.

Alternatively or additionally to any of the embodiments above, processing the imaging data with a processor to generate a longitudinal section view of a blood vessel includes identifying a secondary reference point.

Alternatively or additionally to any of the embodiments above, processing the imaging data with a processor to generate a longitudinal section view of a blood vessel includes processing the imaging data prior to treating the blood vessel with a stent.

Alternatively or additionally to any of the embodiments above, wherein processing the imaging data with a processor to generates a longitudinal section view of a blood vessel includes processing the imaging data after treating the blood vessel with a stent.

An intravascular imaging system is disclosed. The intravascular imaging system comprises: a catheter including an imaging device; a processor coupled to the catheter, the processor configured to process imaging data received from the imaging device; wherein the processor is configured to generate a longitudinal section view of a blood vessel from the imaging data received from the imaging device; wherein the processor is configured to identify a minimum stent area along the longitudinal section view of the blood vessel, a distal reference point, and a proximal reference; and a display unit coupled to the processor, the display unit being configured to show a display including the longitudinal section view of the blood vessel.

Alternatively or additionally to any of the embodiments above, the display includes an indicator of the minimum stent area.

Alternatively or additionally to any of the embodiments above, the indicator of the minimum stent area is movable along the longitudinal section view of the blood vessel by a user.

Alternatively or additionally to any of the embodiments above, the display includes an indicator of the distal reference point.

Alternatively or additionally to any of the embodiments above, the indicator of the distal reference point is movable along the longitudinal section view of the blood vessel by a user.

Alternatively or additionally to any of the embodiments above, the display includes an indicator of the proximal reference point.

Alternatively or additionally to any of the embodiments above, the indicator of the proximal reference point is movable along the longitudinal section view of the blood vessel by a user.

Alternatively or additionally to any of the embodiments above, the processor is configured to identify a second minimum stent area along the longitudinal section view of the blood vessel.

Alternatively or additionally to any of the embodiments above, the processor is configured to identify a secondary reference point along the longitudinal section view of the blood vessel.

An intravascular imaging system is disclosed. The intravascular imaging system comprises: an imaging catheter; a processor coupled to the catheter, the processor configured to process imaging data received from the imaging catheter; wherein the processor is configured to generate a longitudinal section view of a blood vessel from the imaging data; wherein the processor is configured to identify a minimum stent area position along the longitudinal section view of the blood vessel, a distal reference point disposed distal of the minimum stent area position, and a proximal reference disposed proximal of the minimum stent area position; a display unit coupled to the processor, the display unit being configured to show a display; wherein the display includes the longitudinal section view of the blood vessel; and wherein the display includes an indicator of the minimum stent area position, an indicator of the distal reference point, and an indicator of the proximal reference point along the longitudinal section view of the blood vessel.

Alternatively or additionally to any of the embodiments above, the indicator of the minimum stent area position is movable along the longitudinal section view of the blood vessel by a user.

Alternatively or additionally to any of the embodiments above, the indicator of the distal reference point is movable along the longitudinal section view of the blood vessel by a user.

Alternatively or additionally to any of the embodiments above, the indicator of the proximal reference point is movable along the longitudinal section view of the blood vessel by a user.

Alternatively or additionally to any of the embodiments above, the processor is configured to identify a second minimum stent area along the longitudinal section view of the blood vessel.

Alternatively or additionally to any of the embodiments above, the processor is configured to identify a secondary reference point along the longitudinal section view of the blood vessel.

A method for processing imaging data is disclosed. The method comprises: obtaining imaging data from an imaging catheter; processing the imaging data with a processor to generate a longitudinal section view of a blood vessel; wherein processing the imaging data includes identifying a minimum stent area position of the blood vessel along the longitudinal section view of the blood vessel, identifying a distal reference point of the blood vessel along the longitudinal section view of the blood vessel, and identifying a proximal reference point of the blood vessel along the longitudinal section view of the blood vessel; modifying one or more of the minimum stent area position, the distal reference point, and the proximal reference point to a secondary position; and affirming the secondary position.

Alternatively or additionally to any of the embodiments above, modifying one or more of the minimum stent area position, the distal reference point, and the proximal reference point to a secondary position includes translating a bookmark corresponding to the minimum stent area position, the distal reference point, or the proximal reference point along the longitudinal section view of the blood vessel to the secondary position.

Alternatively or additionally to any of the embodiments above, processing the imaging data with a processor to generate a longitudinal section view of a blood vessel includes identifying a secondary reference point.

Alternatively or additionally to any of the embodiments above, processing the imaging data with a processor to generate a longitudinal section view of a blood vessel includes processing the imaging data prior to treating the blood vessel with a stent.

Alternatively or additionally to any of the embodiments above, processing the imaging data with a processor to generates a longitudinal section view of a blood vessel includes processing the imaging data after treating the blood vessel with a stent.

Alternatively or additionally to any of the embodiments above, further comprising an indicator or graphic depicting a suitable percent stent expansion for treating the blood vessel.

Alternatively or additionally to any of the embodiments above, the suitable percent stent expansion is determined from the minimum stent area.

Alternatively or additionally to any of the embodiments above, the suitable percent stent expansion is determined by comparing the minimum stent area to the diameter of the blood vessel at the proximal reference point.

Alternatively or additionally to any of the embodiments above, the suitable percent stent expansion is determined by comparing the minimum stent area to the diameter of the blood vessel at the distal reference point.

Alternatively or additionally to any of the embodiments above, the suitable percent stent expansion is determined by comparing the minimum stent area to the average of the diameters of the blood vessel at the proximal and distal reference points.

An intravascular imaging system is disclosed. The intravascular imaging system comprises: a catheter including an imaging device; a processor coupled to the catheter, the processor configured to process imaging data received from the imaging device; wherein the processor is configured to generate a vessel profile view of a blood vessel from the imaging data received from the imaging device; wherein the processor is configured to identify a minimum stent area along the vessel profile view of the blood vessel, a distal reference point, and a proximal reference; and a display unit coupled to the processor, the display unit being configured to show a display including the vessel view of the blood vessel.

Alternatively or additionally to any of the embodiments above, further comprising an indicator or graphic depicting a suitable percent stent expansion for treating the blood vessel.

Alternatively or additionally to any of the embodiments above, the suitable percent stent expansion is determined from the minimum stent area.

Alternatively or additionally to any of the embodiments above, the suitable percent stent expansion is determined by comparing the minimum stent area to the diameter of the blood vessel at the proximal reference point.

Alternatively or additionally to any of the embodiments above, the suitable percent stent expansion is determined by comparing the minimum stent area to the diameter of the blood vessel at the distal reference point.

Alternatively or additionally to any of the embodiments above, the suitable percent stent expansion is determined by comparing the minimum stent area to the average of the diameters of the blood vessel at the proximal and distal reference points.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 schematically depicts an example intravascular imaging system.
FIG. 2 is a perspective view of an example intravascular imaging catheter system.
FIG. 3 is a side view of a portion of an example intravascular imaging catheter system.
FIG. 4 shows an example display.
FIG. 5 depicts a flow chart for an example process.
FIG. 6 depicts a flow chart for an example process.
FIG. 7 depicts a flow chart for an example process.
FIG. 8 shows an example display.
FIG. 9 shows an example display.
FIG. 10 shows an example display.
FIG. 11 shows an example display.
FIG. 12 shows an example display.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the spirit and scope of the disclosure.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

Intravascular imaging devices insertable into patients have proven diagnostic capabilities for a variety of diseases and disorders. For example, intravascular ultrasound ("IVUS") imaging systems and/or optical coherence tomography ("OCT") imaging systems may be used as an imaging modality for diagnosing blocked blood vessels and providing information to aid medical practitioners in selecting and placing stents and other devices to restore or increase blood flow. IVUS/OCT imaging systems may also be used to diagnose atheromatous plaque build-up at particular locations within blood vessels. IVUS/OCT imaging systems may also be used to determine the existence of an intravascular obstruction or stenosis, as well as the nature and degree of the obstruction or stenosis. IVUS/OCT imaging systems may also be used to visualize segments of a vascular system that may be difficult to visualize using other intravascular imaging techniques, such as angiography, due to, for example, movement (e.g., a beating heart) or obstruction by one or more structures (e.g., one or more blood vessels not desired to be imaged). IVUS/OCT imaging systems may also be used to monitor or assess ongoing intravascular treatments, such as angiography and stent placement in real (or almost real) time. Moreover, IVUS/OCT imaging systems may be used to monitor one or more heart chambers.

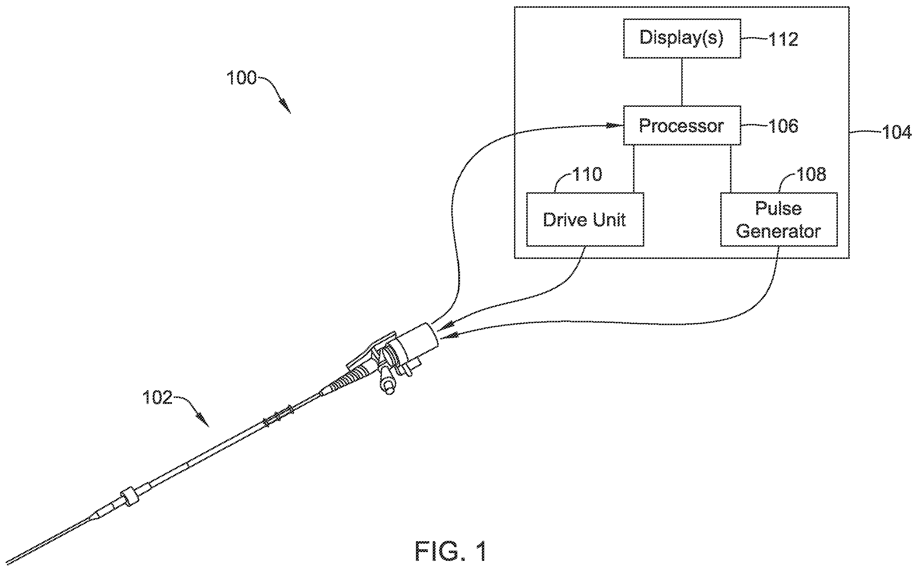

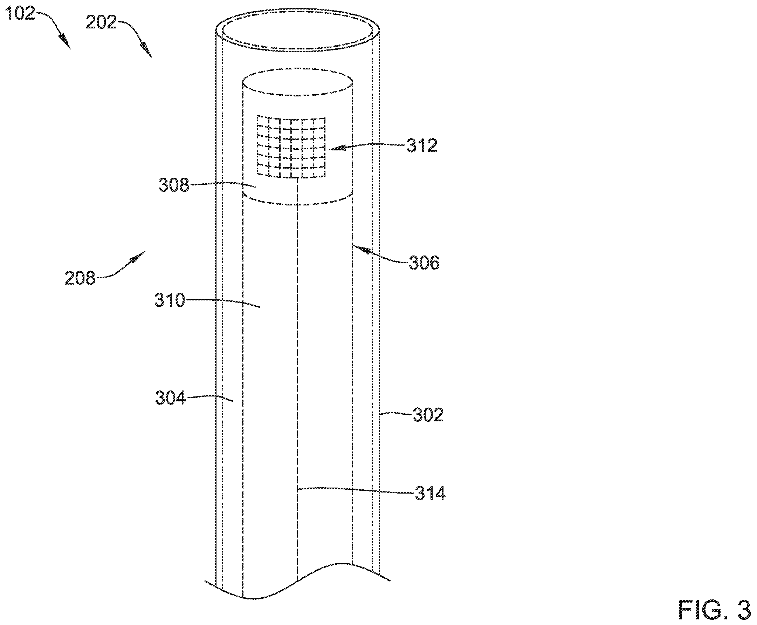

FIG. 1 illustrates schematically an example intravascular imaging system 100. In this example, the intravascular imaging system 100 takes the form of an IVUS imaging system. However, other imaging systems are contemplated including optical coherence tomography imaging systems. The IVUS imaging system 100 includes a catheter 102 that is coupleable to a processing unit or control module 104. The control module 104 may include, for example, a processor 106, a pulse generator 108, a drive unit 110, and one or more displays or display units 112. In some instances, the pulse generator 108 forms electric pulses that may be input to one or more transducers (312 in FIG. 3) disposed in the catheter 102.

For the purposes of this disclosure, the term "display" may either refer to an electronic device (e.g., such as a monitor, etc.) used for the visual representation of data or to the visual representation of data itself. In other words, the term "display" may refer to a hardware device for displaying data or the term "display" may refer to the data displayed on the hardware device. In some cases, it may be helpful to use the phrase "display unit" when referring to the hardware component and the term "display" when referring to the visual/data component shown on the display unit. However, such terminology need not be strictly adhered to in this disclosure or in the art in general. One of skill in the art would be able to discern when the term "display" is referring to a hardware component and when the term "display" is referring to the visual/data component.

In some instances, mechanical energy from the drive unit 110 may be used to drive an imaging core (306 in FIG. 3) disposed in the catheter 102. In some instances, electric signals transmitted from the one or more transducers (312 in FIG. 3) may be input to the processor 106 for processing. In some instances, the processed electric signals from the one or more transducers (312 in FIG. 3) can be displayed as one or more images on the one or more display units 112. For example, a scan converter can be used to map scan line samples (e.g., radial scan line samples, or the like) to a two-dimensional Cartesian grid to display the one or more images on the one or more display units 112.

In some instances, the processor 106 may also be used to control the functioning of one or more of the other components of the control module 104. For example, the processor 106 may be used to control at least one of the frequency or duration of the electrical pulses transmitted from the pulse generator 108, the rotation rate of the imaging core (306 in FIG. 3) by the drive unit 110, the velocity or length of the pullback of the imaging core (306 in FIG. 3) by the drive unit 110, or one or more properties of one or more images formed on the one or more display units 112.

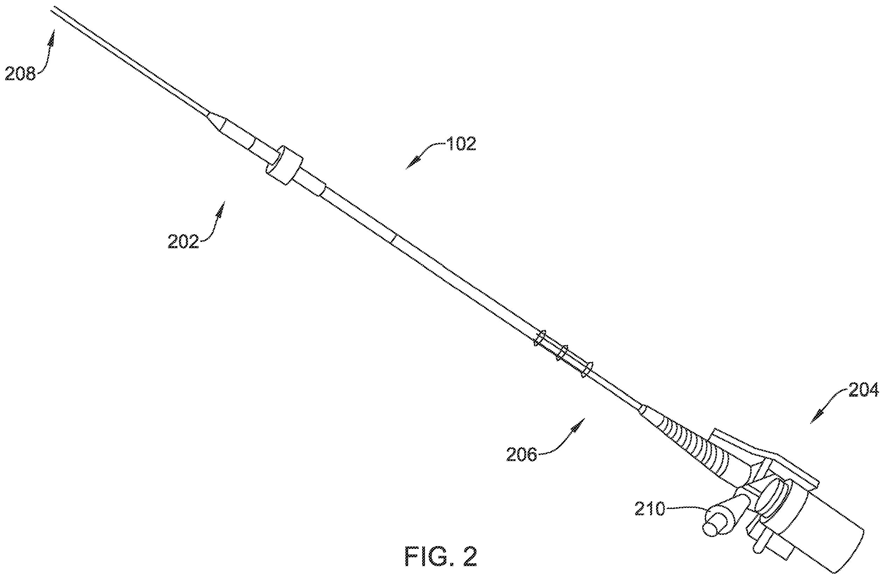

FIG. 2 is a schematic side view of one embodiment of the catheter 102 of the IVUS imaging system (100 in FIG. 1). The catheter 102 includes an elongated member 202 and a hub 204. The elongated member 202 includes a proximal end 206 and a distal end 208. In FIG. 2, the proximal end 206 of the elongated member 202 is coupled to the catheter hub 204 and the distal end 208 of the elongated member is configured and arranged for percutaneous insertion into a patient. Optionally, the catheter 102 may define at least one flush port, such as flush port 210. The flush port 210 may be defined in the hub 204. The hub 204 may be configured and arranged to couple to the control module (104 in FIG 1). In some instances, the elongated member 202 and the hub 204 are formed as a unitary body. In other instances, the elongated member 202 and the catheter hub 204 are formed separately and subsequently assembled together.

FIG. 3 is a schematic perspective view of one embodiment of the distal end 208 of the elongated member 202 of the catheter 102. The elongated member 202 includes a sheath 302 with a longitudinal axis (e.g., a central longitudinal axis extending axially through the center of the sheath 302 and/or the catheter 102) and a lumen 304. An imaging core 306 is disposed in the lumen 304. The imaging core 306 includes an imaging device 308 coupled to a distal end of a driveshaft 310 that is rotatable either manually or using a computer-controlled drive mechanism. One or more transducers 312 may be mounted to the imaging device 308 and employed to transmit and receive acoustic signals. The sheath 302 may be formed from any flexible, biocompatible material suitable for insertion into a patient. Examples of suitable materials include, for example, polyethylene, polyurethane, plastic, spiral-cut stainless steel, nitinol hypotube, and the like or combinations thereof.

In some instances, for example as shown in Figure 3, an array of transducers 312 are mounted to the imaging device 308. Alternatively, a single transducer may be employed. Any suitable number of transducers 312 can be used. For example, there can be two, three, four, five, six, seven, eight, nine, ten, twelve, fifteen, sixteen, twenty, twenty-five, fifty, one hundred, five hundred, one thousand, or more transducers. As will be recognized, other numbers of transducers may also be used. When a plurality of transducers 312 are employed, the transducers 312 can be configured into any suitable arrangement including, for example, an annular arrangement, a rectangular arrangement, or the like.

The one or more transducers 312 may be formed from materials capable of transforming applied electrical pulses to pressure distortions on the surface of the one or more transducers 312, and vice versa. Examples of suitable materials include piezoelectric ceramic materials, piezocomposite materials, piezoelectric plastics, barium titanates, lead zirconate titanates, lead metaniobates, polyvinylidene fluorides, and the like. Other transducer technologies include composite materials, single-crystal composites, and semiconductor devices (e.g., capacitive micromachined ultrasound transducers ("cMUT"), piezoelectric micromachined ultrasound transducers ("pMUT"), or the like).

The pressure distortions on the surface of the one or more transducers 312 form acoustic pulses of a frequency based on the resonant frequencies of the one or more transducers 312. The resonant frequencies of the one or more transducers 312 may be affected by the size, shape, and material used to form the one or more transducers 312. The one or more transducers 312 may be formed in any shape suitable for positioning within the catheter 102 and for propagating acoustic pulses of a desired frequency in one or more selected directions. For example, transducers may be disc-shaped, block-shaped, rectangular-shaped, oval-shaped, and the like. The one or more transducers may be formed in the desired shape by any process including, for example, dicing, dice and fill, machining, microfabrication, and the like.

As an example, each of the one or more transducers 312 may include a layer of piezoelectric material sandwiched between a matching layer and a conductive backing material formed from an acoustically absorbent material (e.g., an epoxy substrate with tungsten particles). During operation, the piezoelectric layer may be electrically excited to cause the emission of acoustic pulses.

The one or more transducers 312 can be used to form a radial cross-sectional image of a surrounding space. Thus, for example, when the one or more transducers 312 are disposed in the catheter 102 and inserted into a blood vessel of a patient, the one more transducers 312 may be used to form an image of the walls of the blood vessel and tissue surrounding the blood vessel.

The imaging core 306 is rotated about the longitudinal axis of the catheter 102. As the imaging core 306 rotates, the one or more transducers 312 emit acoustic signals in different radial directions (e.g., along different radial scan lines). For example, the one or more transducers 312 can emit acoustic signals at regular (or irregular) increments, such as 256 radial scan lines per revolution, or the like. It will be understood that other numbers of radial scan lines can be emitted per revolution, instead.

When an emitted acoustic pulse with sufficient energy encounters one or more medium boundaries, such as one or more tissue boundaries, a portion of the emitted acoustic pulse is reflected back to the emitting transducer as an echo pulse. Each echo pulse that reaches a transducer with sufficient energy to be detected is transformed to an electrical signal in the receiving transducer. The one or more transformed electrical signals are transmitted to the control module (104 in FIG. 1) where the processor 106 processes the electrical-signal characteristics to form a displayable image of the imaged region based, at least in part, on a collection of information from each of the acoustic pulses transmitted and the echo pulses received. In some instances, the rotation of the imaging core 306 is driven by the drive unit 110 disposed in the control module (104 in FIG. 1). In alternate embodiments, the one or more transducers 312 are fixed in place and do not rotate. In which case, the driveshaft 310 may, instead, rotate a mirror that reflects acoustic signals to and from the fixed one or more transducers 312.

When the one or more transducers 312 are rotated about the longitudinal axis of the catheter 102 emitting acoustic pulses, a plurality of images can be formed that collectively form a radial cross-sectional image (e.g., a tomographic image) of a portion of the region surrounding the one or more transducers 312, such as the walls of a blood vessel of interest and tissue surrounding the blood vessel. The radial cross-sectional image can, optionally, be displayed on one or more display units 112. The at least one of the imaging core 306 can be either manually rotated or rotated using a computer-controlled mechanism.

The imaging core 306 may also move longitudinally along the blood vessel within which the catheter 102 is inserted so that a plurality of cross-sectional images may be formed along a longitudinal length of the blood vessel. During an imaging procedure the one or more transducers 312 may be retracted (e.g., pulled back) along the longitudinal length of the catheter 102. The catheter 102 can include at least one telescoping section that can be retracted during pullback of the one or more transducers 312. In some instances, the drive unit 110 drives the pullback of the imaging core 306 within the catheter 102. The drive unit 110 pullback distance of the imaging core can be any suitable distance including, for example, at least 5 cm, 10 cm, 15 cm, 20 cm, 25 cm, or more. The entire catheter 102 can be retracted during an imaging procedure either with or without the imaging core 306 moving longitudinally independently of the catheter 102.

A stepper motor may, optionally, be used to pull back the imaging core 306. The stepper motor can pull back the imaging core 306 a short distance and stop long enough for the one or more transducers 306 to capture an image or series of images before pulling back the imaging core 306 another short distance and again capturing another image or series of images, and so on.

The quality of an image produced at different depths from the one or more transducers 312 may be affected by one or more factors including, for example, bandwidth, transducer focus, beam pattern, as well as the frequency of the acoustic pulse. The frequency of the acoustic pulse output from the one or more transducers 312 may also affect the penetration depth of the acoustic pulse output from the one or more transducers 312. In general, as the frequency of an acoustic pulse is lowered, the depth of the penetration of the acoustic pulse within patient tissue increases. In some instances, the IVUS imaging system 100 operates within a frequency range of 5 MHz to 100 MHz.

One or more conductors 314 can electrically couple the transducers 312 to the control module 104 (see, for example, FIG. 1). In which case, the one or more conductors 314 may extend along a longitudinal length of the rotatable driveshaft 310.

The catheter 102 with one or more transducers 312 mounted to the distal end 208 of the imaging core 308 may be inserted percutaneously into a patient via an accessible blood vessel, such as the femoral artery, femoral vein, or jugular vein, at a site remote from the selected portion of the selected region, such as a blood vessel, to be imaged. The catheter 102 may then be advanced through the blood vessels of the patient to the selected imaging site, such as a portion of a selected blood vessel.

An image or image frame ("frame") can be generated each time one or more acoustic signals are output to surrounding tissue and one or more corresponding echo signals are received by the imager 308 and transmitted to the processor 106. Alternatively, an image or image frame can be a composite of scan lines from a full or partial rotation of the imaging core or device. A plurality (e.g., a sequence) of frames may be acquired over time during any type of movement of the imaging device 308. For example, the frames can be acquired during rotation and pullback of the imaging device 308 along the target imaging location. It will be understood that frames may be acquired both with or without rotation and with or without pullback of the imaging device 308. Moreover, it will be understood that frames may be acquired using other types of movement procedures in addition to, or in lieu of, at least one of rotation or pullback of the imaging device 308.

In some instances, when pullback is performed, the pullback may be at a constant rate, thus providing a tool for potential applications able to compute longitudinal vessel/plaque measurements. In some instances, the imaging device 308 is pulled back at a constant rate of about 0.3-0.9 mm/s or about 0.5-.8 mm/s. In some instances, the imaging device 308 is pulled back at a constant rate of at least 0.3 mm/s. In some instances, the imaging device 308 is pulled back at a constant rate of at least 0.4 mm/s. In some instances, the imaging device 308 is pulled back at a constant rate of at least 0.5 mm/s. In some instances, the imaging device 308 is pulled back at a constant rate of at least 0.6 mm/s. In some instances, the imaging device 308 is pulled back at a constant rate of at least 0.7 mm/s. In some instances, the imaging device 308 is pulled back at a constant rate of at least 0.8 mm/s.

In some instances, the one or more acoustic signals are output to surrounding tissue at constant intervals of time. In some instances, the one or more corresponding echo signals are received by the imager 308 and transmitted to the processor 106 at constant intervals of time. In some instances, the resulting frames are generated at constant intervals of time.

At least some conventional IVUS imaging systems display only a single (e.g., cross-sectional, longitudinal, or the like) image during, or after, an IVUS procedure, such as a pull-back procedure. It may, however, be useful to concurrently display, in real-time during the IVUS procedure (e.g., a pull-back procedure), at least two images, such as the most recently processed image and a previously-obtained image that has some particular or selected image characteristic (e.g., maximum or minimum lumen area or diameter).

Some diagnostic and/or therapeutic interventions may include the analysis of images generated by the IVUS imaging system. This analysis, however, may require a substantial amount of training/experience in order to efficiently interpret the images. Furthermore, due to the frequent presence of speckles on IVUS images, automated analysis and/or evaluation may also be challenging. Disclosed herein are methods for processing and/or analyzing images such as images generated with/by an IVUS imaging system. Such methods may utilize machine learning, artificial intelligence, deep neural networks, and/or the like to improve the processing and/or analysis of images generated with/by an IVUS imaging system.

The processes/methods disclosed herein may include the generation and/or collection of images of a blood vessel (e.g., IVUS images, cross-sectional images, etc. generated via an IVUS pullback procedure). The generated/collected images may be subjected to processing and/or segmentation of the images using deep learning networks (e.g., deep neural networks such as the U-Net deep neural network) to obtain image segmentation for quantitative analysis and image classification for automated identification of lesion type, stent detection, identification of the lumen border, identification of the lumen dimensions, identification of the minimum lumen area (MLA), identification of the media border (e.g., identification of a media border for media within the blood vessel), identification of the media dimensions, identification of the calcification angle/arc, identification of the calcification coverage, identification of the lesion types, combinations thereof, and/or the like. In addition to identifying such border/dimension, the output may be displayed on a display unit in a suitable format (e.g., graphically, numerically, as a real or schematic image, with words or symbols, etc.). In some instances, multiple images of an IVUS pullback or "run" may be analyzed. The output of this run analysis may include a lumen profile (e.g., including, for example, a longitudinal section or "long view"), a vessel profile (e.g., including, for example, a longitudinal section or "long view"), a representation (e.g., visualization or image, numerical visualization, graphical visualization, and/or the like) of the calcification length, the depiction/display of reference frames (e.g., such as the minimal lumen area or "MLA", the minimal stent area or "MSA", or the like), a representation (e.g., visualization or image, numerical visualization, graphical visualization, and/or the like) of side branch location, a representation (e.g., visualization or image, numerical visualization, graphical visualization, and/or the like) of the distance between two frames of interest, a representation (e.g., visualization or image, numerical visualization, graphical visualization, and/or the like) of stent extension, combinations thereof, and/or the like. This may also include analyzing the images with a deep neural network (e.g., such as UNet deep neural network) and/or machine learning and/or artificial intelligence.

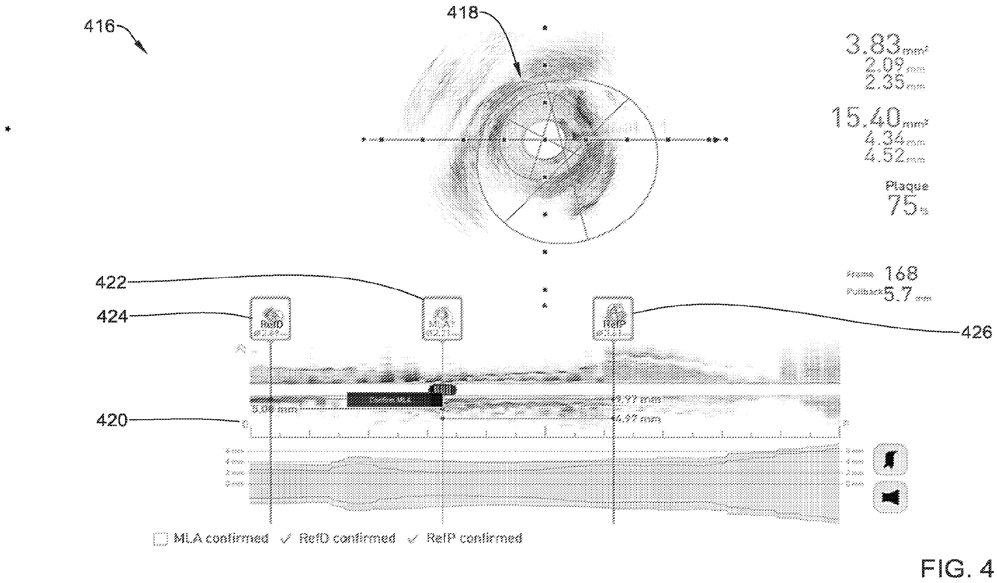

FIG. 4 shows an example display 416, which may be displayed on the display/display unit 112. Among other things, the display 416 includes a transverse cross-sectional image 418 of an example blood vessel and a longitudinal section image/display 420 of the blood vessel. In this example, the processor 106 may be configured to automatically identify a minimum lumen area (MLA) of the blood vessel and then output/display an indicator or bookmark corresponding to the MLA 422 along the longitudinal section display 420. This may include the use of a deep neural network (e.g., such as UNet deep neural network) and/or machine learning and/or artificial intelligence. The processor 106 may also be configured to identify a proximal reference point and a distal reference point. This may also include the use of a deep neural network (e.g., such as UNet deep neural network) and/or machine learning and/or artificial intelligence. The processor 106 may also be configured to output/display an indicator or bookmark corresponding to the distal reference point 424 and an indicator or bookmark corresponding to the proximal reference point 426 along the longitudinal section display 420. In general, the proximal and distal reference points may be chosen as potential locations where the ends of a stent may be situated in order to treat the lesions defined about the MLA. Thus, the indicators 422, 424, 426 may help a clinician locate a potential deployment position for a stent.

It can be appreciated that the indicator 422 may be used to denote MLA in pre-percutaneous coronary intervention (PCI) IVUS pullbacks/runs. In post-PCI, the indicator 422 may correspond to the minimum stent area (MSA). Thus, in post-PCI pullbacks/runs, the indicator 422 may bear the label MSA.

In addition, the longitudinal section display 420 may provide useful information to a clinician. In some instances, the longitudinal section display 420 may include or, alternatively, be replaced by a vessel profile view, which provides information on the vessel including a representation (e.g., visualization or image, numerical visualization, graphical visualization, and/or the like) of the calcification angle/arc, a representation (e.g., visualization or image, numerical visualization, graphical visualization, and/or the like) of the calcification length, the depiction/display of reference frames (e.g., such as MLA, MSA, and/or the like), a representation (e.g., visualization or image, numerical visualization, graphical visualization, and/or the like) of side branch location, a representation (e.g., visualization or image, numerical visualization, graphical visualization, and/or the like) of the distance between two frames of interest, a representation (e.g., visualization or image, numerical visualization, graphical visualization, and/or the like) of stent extension, combinations thereof, and/or the like. In some instances, the display 416 may include the longitudinal section display 420, the vessel profile display, or both.

In some instances, the processor 106 may be configured to output/display another indicator or bookmark other than just the indicators 422, 424, 426. This may include additional automatic indicators (e.g., additional MLA indicators and/or additional/secondary reference point indicators), which may appear along the longitudinal cross-sectional display 420 without any user intervention. In some of these and in other instances, manual indicators or bookmarks may be added by the user. For example, a user may wish to add an indicator or bookmark (e.g., a secondary indicator or bookmark) to the longitudinal section display 420 and by using a suitable input device (e.g., a mouse, keyboard, touch pad, a finger or gesture on a touchscreen, etc.), the user can add the additional indicator.

While the processor 106 may provide a reliable level of precision and accuracy when identifying the MLA, the distal reference point, and the proximal reference point, and/or identifying other features of a blood vessel, a clinician may still wish to review the locations of these points and, if desired, confirm and/or modify the location of these points.

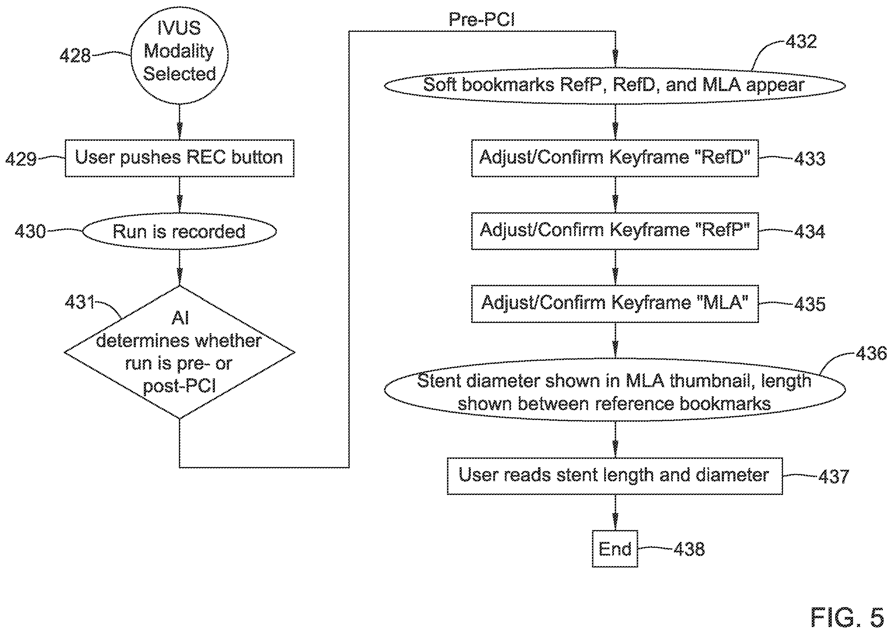

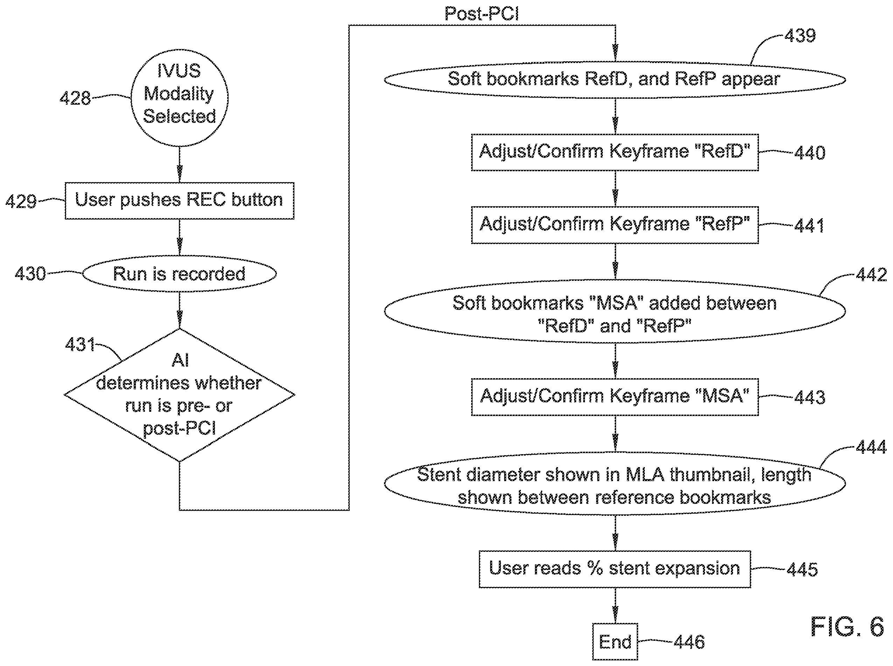

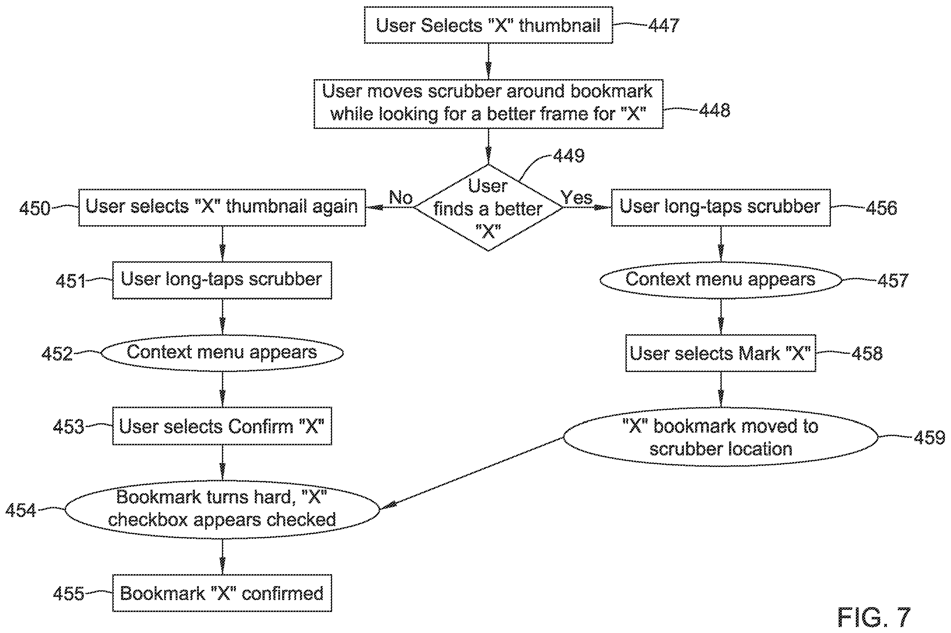

FIGS. 5-7 are flow charts displaying the workflow for pre-PCI and post-PCI IVUS pullbacks/runs and how the indicators 422, 424, 426 may be selected, altered, and confirmed on the display 416. In FIG. 5, the flowchart corresponds to process for an IVUS pullback prior to a PCI. In FIG. 6, the flowchart corresponds to a process for an IVUS pullback after a PCI. Finally, FIG. 7 is a flowchart demonstrating the steps for adjusting/confirming the indicators 422, 424, 426.

Turning now to FIG. 5, the process may include selecting the IVUS modality (e.g., on the control module 104) at box 428. This may include selecting the IVUS modality from a list of possible modalities using a suitable user input (e.g., such as a mouse, keyboard, touchpad, touch screen on the display/display unit 112, etc.). At box 429, the user may activate the recording feature by pushing the record (REC) button, which initiates the recording. Box 430 corresponds to the IVUS pullback/run being recorded. The processor 106 may utilize artificial intelligence that is able to determine whether the IVUS pullback/run is taking place pre-PCI or post-PCI as shown at box 431.

For the pre-PCI run at box 432, the processor may utilize artificial intelligence that can automatically place the indicators 422, 424, 426, for example as soft bookmarks corresponding to the proximal reference point (RefP), the distal reference point (ReID) and the MLA on the longitudinal section display 420. The indicators/bookmarks 422, 424, 426 are deemed "soft" in that the user can still review the position of the indicators/bookmarks 422, 424, 426 and, if desired, make any desired changes prior to locking in/confirming or otherwise making the indicators/bookmarks 422, 424, 426 "hard". At boxes 433, 434, 435, a user can adjust/confirm the indicators/bookmarks for RefD, RefP and MLA, respectively. The process for adjusting/confirming the indicators/bookmarks is represented in FIG. 7.

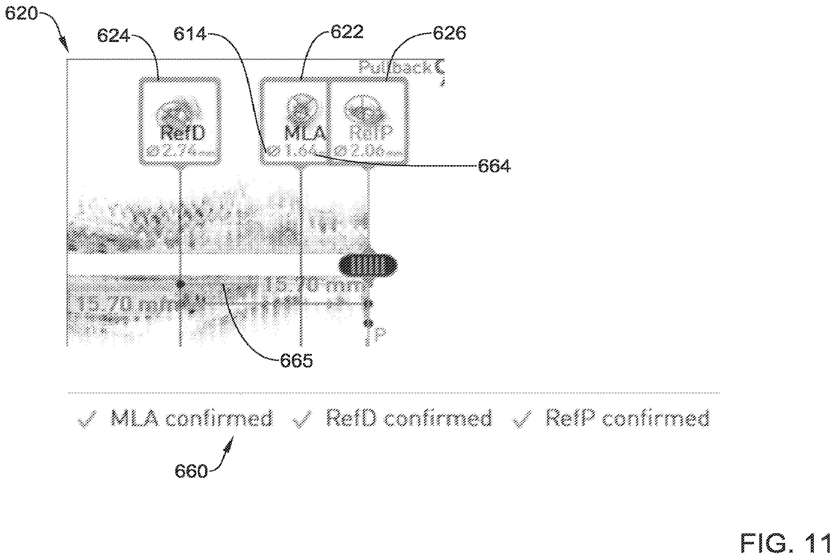

At box 436, the stent diameter (e.g., which may be displayed adjacent to the MLA indicator 422; please see FIG. 11) and proposed stent lengths for an intervention (e.g., which may be displayed using bars disposed between the distal reference point indicator 424 and the proximal reference point indicator 426; please see FIG. 11). The displayed values for "stent diameter" may be actual diameter of the vessel at the MLA indicator 422 (e.g., which may be used to determine a suitable stent diameter size that can be used during an intervention) or a proposed stent diameter to be used for the intervention. In some instances, a user may be able to toggle between the actual diameter and the proposed stent diameter. Similarly, the values for "stent length" may be actual length between the reference points (e.g., which may be used to determine a suitable stent length that can be used during an intervention) or a proposed stent length to be used for the intervention. In some instances, a user may be able to toggle between the actual length and the proposed stent length. A user may review the stent diameter and length information at box 437 and then complete the process at box 438.

For the post-PCI run at box 439 in FIG. 6, the processor may utilize artificial intelligence that can place the indicators 422, 424, 426, for example as keyframes and/or soft bookmarks corresponding to the proximal reference point (RefP), the distal reference point (RefD) and the minimum stent area (MSA) on the longitudinal section display 420. It is noted that in this description of post-PCI runs, the reference number 422 is used for the MSA indicator and that this indicator 422 can be output to the display 416. At boxes 440, 441, 442, a user can adjust/confirm the indicators/bookmarks for RefD, RefP and MSA, respectively. The process for adjusting/confirming the indicators/bookmarks is represented in FIG. 7.

At box 444, the stent diameter (e.g., which may be displayed adjacent to the MLA/MSA indicator 422; please see FIG. 11) and proposed stent lengths for an intervention (e.g., which may be displayed using bars disposed between the distal reference point indicator 424 and the proximal reference point indicator 426; please see FIG. 11). A user may review the extent of stent expansion (e.g., percent stent expansion) at box 445 and then complete the process at box 446.

FIG. 7 is a flowchart depicting the process for adjusting/confirming the indicators 422, 424, 426. FIG. 7 expands upon boxes 433, 434, 435 in FIG. 5 and boxes 440, 441, 443 in FIG. 6. In this flowchart, "X" is a generic designation corresponding to any one of the given indicators 422, 424, 426 and the process can be utilized for adjusting/confirming any one or all of given indicators. At box 447, a user may select a bookmark or indicator 422, 424, 426. At box 448, the user may move the scrubber to look for a more desirable location for the particular bookmark or indicator 422, 424, 426. When doing so, the selected indicator/bookmark 422, 424, 426 moves/translates along the longitudinal section display 420. It can be appreciated that each of the indicators 422, 424, 426 are independently movable along the longitudinal section display 420. At box 449, the user may determine if a more desirable location has been located. If not, user may select the particular bookmark or indicator 422, 424, 426 (box 450), long tap/hold the scrubber (box 451), which causes a context menu to appear at box 452. Within the context menu, the user may confirm the location of the indicator (box 453), which makes the indicator/bookmark "hard" and a checkbox appears checked for that particular bookmark or indicator 422, 424, 426 (box 454). This confirms the particular bookmark or indicator 422, 424, 426 (box 455).

If at box 449 a more desirable location for the particular bookmark or indicator 422, 424, 426 is found, the user may long tap/hold the scrubber (box 456) while the scrubber is at the alternative/secondary location, causing a context menu to appear (box 457). The user may select to mark the new location (box 458) and the particular bookmark or indicator 422, 424, 426 may be moved to the new/secondary/alternative location (box 459). This makes the indicator/bookmark "hard" and a checkbox appears checked for that particular bookmark or indicator 422, 424, 426 (box 454) and the particular bookmark or indicator 422, 424, 426 is then confirmed (box 455).

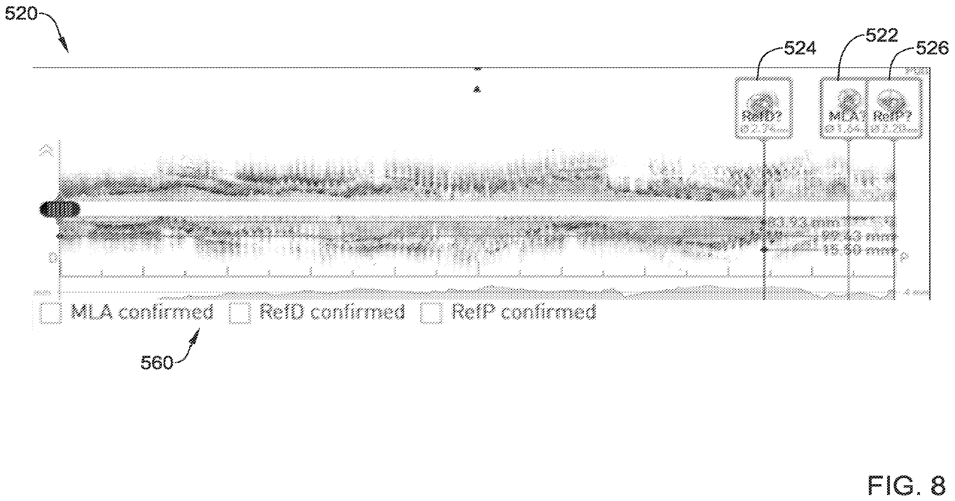

FIGS. 8-11 show longitudinal section displays (e.g., similar to the longitudinal section display 420 in FIG. 4) at some example points along the processes described with reference to FIGS. 5-7. For example, FIG. 8 illustrates a longitudinal section display 520. Here, "soft" indicators/bookmarks 522, 524, 526 are shown along the longitudinal section display 520, corresponding to box 432 in FIG. 5 and/or box 439 in FIG. 6 (noting that in a post-PCT run, the indicator 522 may be an indicator of MSA). Also shown in FIG. 8 is a menu 460 showing that none of the indicators 522, 524, 526 are confirmed (e.g., by virtue of the checkbox next to the given label not being selected).

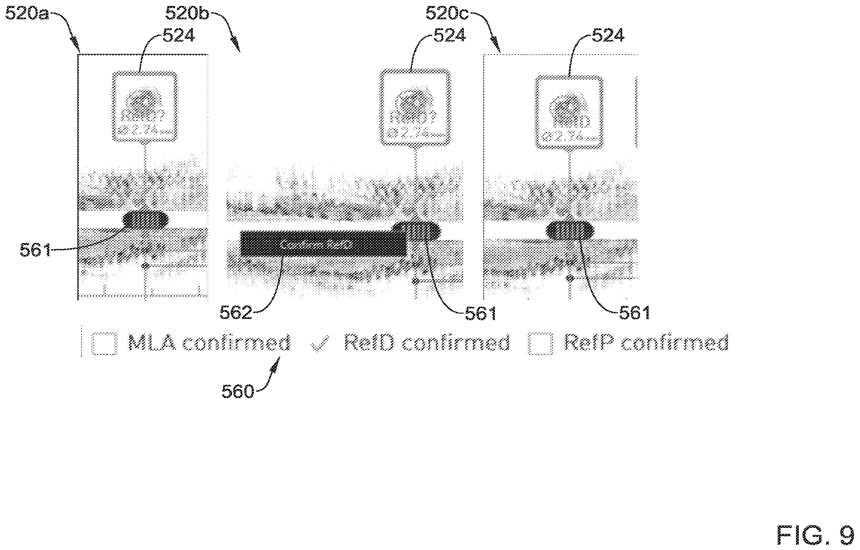

A process for adjusting/confirming a particular indicator is shown in FIG. 9. In this example, the process includes adjusting/confirming the indicator 524 for the distal reference point (ReID). In FIG. 9, three separate depictions of the longitudinal section display 520 are shown, namely depictions 520a, 520b, 520c. In depiction 520a, a user may select the indicator/bookmark 524 (e.g., box 447 in FIG. 7) by touching the scrubber 561 (e.g., when using a touch screen display, the user may use the finger or a suitable input device). The scrubber 561 may be moved (e.g., by translating the slider using a finger or suitable input device) to a new location (e.g., box 448 in FIG. 7) as shown in depiction 520b. If the user does not find the new location to be more desirable, the user may long tap/hold the scrubber 561 (e.g., box 451 in FIG. 7) and a context menu 562 may appear (box 452 in FIG. 7). The user may confirm the location (box 453 in FIG. 7) and the indicator/bookmark 524 then becomes "hard" (box 454 in FIG. 7.) and may be confirmed by the check in the menu 560 (box 455 in FIG. 7).

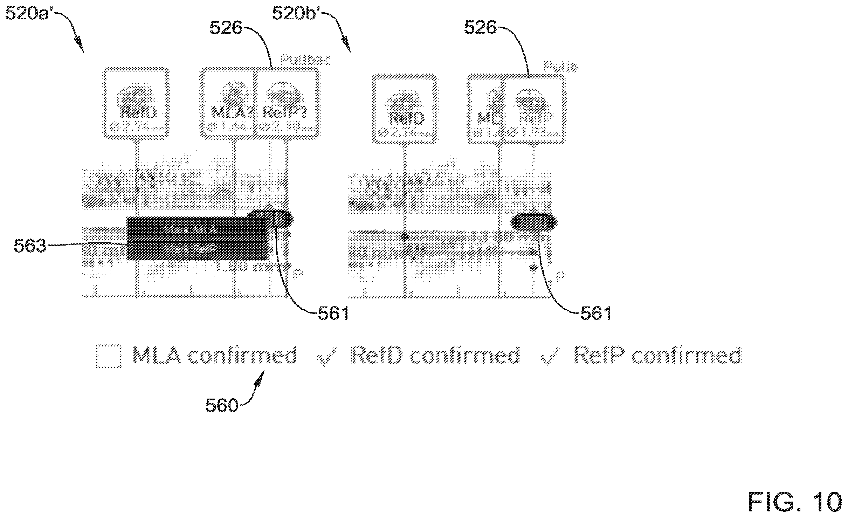

In FIG. 10, the process may be similarly carried out for another one of the indicators/bookmarks, in this example the indicator/bookmark 526 corresponding to the proximal reference point (RefP). In FIG. 10, two separate depictions of the longitudinal section display 520 are shown, namely depictions 520a, 520b. In depiction 520a, a user may select the indicator/bookmark 526 (e.g., box 447 in FIG. 7) by touching the scrubber 561. The scrubber 561 may be moved to a new location (e.g., box 448 in FIG. 7) as shown in depiction 520b. If the user finds the new location to be more desirable, the user may long tap/hold the scrubber 561 (e.g., box 456 in FIG. 7) and a context menu 563 may appear (e.g., box 457 in FIG. 7). The user may mark the new location (box 458 in FIG. 7) and the indicator/bookmark 526 may be moved to the new location (box 459 in FIG. 7). The indicator/bookmark 526 may then become "hard" (box 454 in FIG. 7.) and may be confirmed by the check in the menu 560 (box 455 in FIG. 7).

FIG. 11 illustrates a longitudinal section display 620. In the longitudinal section display 620, the menu 660 shows that each of the indicators/bookmarks are confirmed by the check in the menu 660. Also, as described above, the MLA indicator 622 may also display the diameter at the MLA 664, which may aid a clinician in determining a suitable stent diameter size to be used for an intervention. The distance between indicator 624 and indicator 626 is also shown with a bar 665. The bar may be labeled with the distance between the indicators 624, 626, which may help a clinician determine a suitable stent length that can be used to treat the lesion.

The longitudinal section display 620 and/or other longitudinal section displays disclosed herein may also provide an indicator or graphic depicting percent stent expansion. For example, the processor 106 may be capable of determining a desirable amount/percent of stent expansion suitable for treating the blood vessel based on the MSA alone. Alternatively, the processor 106 may compare the MSA to (a) the diameter of the blood vessel at the proximal reference point, (b) the diameter of the blood vessel at the proximal reference point, or (c) the mean or average of the diameters at the proximal and distal reference points. Based on these comparisons, the indicator or graphic may be disposed adjacent to the longitudinal section display 620 indicating one or more of (a) desired percent stent expansion for a treatment based on MSA alone, (b) desired percent stent expansion for a treatment based on comparing MSA to the diameter of the blood vessel at the proximal reference point, (c) desired percent stent expansion for a treatment based on comparing MSA to the diameter of the blood vessel at the distal reference point, and/or (d) desired percent stent expansion for a treatment based on comparing MSA to the mean or average of the diameters of the blood vessel at the proximal and distal reference points.

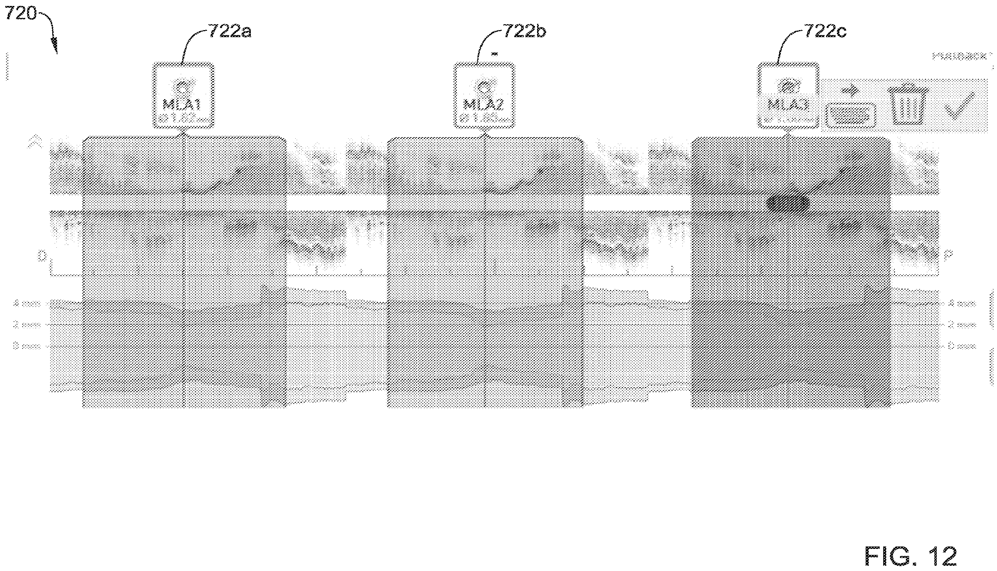

It can be appreciated that some cross-sectional longitudinal displays may include more than one area depicting an indicator/bookmark. For example, FIG. 12 illustrates a cross-sectional longitudinal display 720 that may include multiple indicators 722a, 722b, 722c for MLA. While only shown schematically in FIG. 12, it can be appreciated that each MLA indicator 722a, 722b, 722c may have distal and proximal reference points, etc.

U.S. Patent Application No. 62/906,546.

WO 2021/062006.

U.S. Patent Application No. 63/157,427.

U.S. patent Application No. 63/233,875.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. An intravascular ultrasound, IVUS, imaging system (100), comprising:
a catheter (102) including an elongated member (202) and a hub (204), wherein the elongated member (202) includes a sheath (302) with a longitudinal axis and lumen (304) with an imaging core (306) disposed in the lumen (304), wherein the imaging core (306) includes an imaging device (308) coupled to a distal end of a driveshaft (310) that is rotatable either manually or using a computer-controlled drive mechanism; and
a control module (104) including a processor (106), a pulse generator (108), a drive unit (110) and one or more displays (112);
wherein, in an IVUS run, the imaging core (306) is adapted to move longitudinally along a blood vessel within which the catheter (102) is inserted to form a plurality of cross-sectional images along a longitudinal length of the blood vessel;
wherein the processor (106) is configured to process the cross-sectional images received from the imaging device (308) using deep learning networks to obtain image segmentation for quantitative analysis and image classification for automated identification of one or a combination of: lesion type, stent detection, identification of the lumen border, identification of the lumen dimensions, identification of the minimum lumen area, identification of the media border, identification of the media dimensions, identification of the calcification angle/arc, identification of the calcification coverage, identification of the lesion types;
wherein the processor (106) is further configured to output one or a combination of: a lumen profile including, for example, a longitudinal section, a vessel profile including, for example, a longitudinal section, a representation of the calcification length, the display of reference frames such as the minimal lumen area, MLA, the minimal stent area, MSA, a representation of side branch location, a representation of the distance between two frames of interest, a representation of stent extension;
wherein the processor (106) is configured to determine whether the IVUS run is taking place pre-percutaneous coronary intervention - PCI - or post-PCI using artificial intelligence.

2. The system of claim 1, wherein the processor (106) is further configured to display a transverse cross-sectional image (418) and a longitudinal section image (420) of the blood vessel on the one or more displays (112).

3. The system of claim 2, wherein the processor (106) is further configured to identify a proximal reference point and a distal reference point, and to display an indicator corresponding to the distal reference point (424) and an indicator corresponding to the proximal reference point (426) along the longitudinal section image (420).

4. The system of claim 3, wherein the processor (106) is further configured to allow a user to confirm and/or modify the location of the reference points.

5. The system of any of the previous claims, wherein the processor (106) is configured to process the cross-sectional images received from the imaging device (308) to obtain image segmentation for quantitative analysis and image classification for automated identification of a lesion type;
and wherein the processor (106) is further configured to output a vessel profile.

6. The system of any of the previous claims, wherein the processor (106) is configured to process the cross-sectional images received from the imaging device (308) to obtain image segmentation for quantitative analysis and image classification for automated identification of stent detection;
and wherein the processor (106) is further configured to output a vessel profile.

7. The system of any of the previous claims, wherein the processor (106) is configured to process the cross-sectional images received from the imaging device (308) to obtain image segmentation for quantitative analysis and image classification for automated identification of an identification of the calcification angle/arc;
and wherein the processor (106) is further configured to output a representation of the calcification length.

8. The system of any of the previous claims, wherein the processor (106) is configured to process the cross-sectional images received from the imaging device (308) to obtain image segmentation for quantitative analysis and image classification for automated identification of an identification of the calcification coverage;
and wherein the processor (106) is further configured to output: a representation of the calcification length.

9. The system of any one of claims 1 to 8, wherein the processor (106) is configured, for a pre-PCI IVUS run, to utilize artificial intelligence to automatically place indicators corresponding to a proximal reference point, a distal reference point and the minimal lumen area on the longitudinal section display.

10. The system of any one of claims 1 to 9, wherein the processor (106) is configured to display a proposed stent length and/or a proposed stent diameter for an intervention.

11. The system of claim 10, wherein the displayed proposed stent diameter corresponds to the vessel diameter at the minimal lumen area.

12. The system of any of the previous claims, wherein the processor (106) is configured to determine a desirable amount and/or percent of stent expansion suited for treating the blood vessel based on the minimal lumen area.

## Patentansprüche

1. Intravaskuläres Ultraschall-, IVUS, Bildgebungssystem (100), aufweisend:
einen Katheter (102) mit einem länglichen Element (202) und einer Nabe (204), wobei das längliche Element (202) eine Hülse (302) mit einer Längsachse und einem Lumen (304) aufweist, wobei ein Bildgebungskern (306) im Lumen (304) angeordnet ist, wobei der Bildgebungskern (306) eine Bildgebungsvorrichtung (308) aufweist, die mit einem distalen Ende einer Antriebswelle (310) verbunden ist, die entweder manuell oder unter Verwendung eines computergesteuerten Antriebsmechanismus drehbar ist; und
ein Steuermodul (104) mit einem Prozessor (106), einem Impulsgenerator (108), einer Antriebseinheit (110) und einem oder mehreren Displays (112),
wobei bei einem IVUS-Durchlauf der Bildgebungskern (306) dazu eingerichtet ist, sich in der Längsrichtung entlang eines Blutgefäßes zu bewegen, in das der Katheter (102) eingeführt ist, um eine Mehrzahl von Querschnittsbildern entlang einer Längsausdehnung des Blutgefäßes zu erzeugen,
wobei der Prozessor (106) dafür konfiguriert ist, die von der Bildgebungsvorrichtung (308) empfangenen Querschnittsbilder unter Verwendung von Deep-Learning-Netzwerken zu verarbeiten, um eine Bildsegmentierung für eine quantitative Analyse und eine Bildklassifizierung zum automatisierten Identifizieren eines oder einer Kombination der folgenden Elemente zu erhalten: Läsionstyp, Stent-Erfassung, Identifizieren der Lumengrenze, Identifizieren der Lumenabmessungen, Identifizieren der minimalen Lumenfläche, Identifizieren der Mediagrenze, Identifizieren der Mediaabmessungen, Identifizieren des Verkalkungswinkels/-bogens, Identifizieren der Verkalkungsabdeckung, Identifizieren der Läsionstypen,
wobei der Prozessor (106) ferner dafür konfiguriert ist, eines oder eine Kombination der folgenden Elemente auszugeben: ein Lumenprofil, das beispielsweise einen Längsschnitt aufweist, ein Gefäßprofil, das beispielsweise einen Längsschnitt aufweist, eine Darstellung der Verkalkungslänge, die Anzeige von Referenzbildern wie der minimalen Lumenfläche, MLA, der minimalen Stentfläche, MSA, eine Darstellung der Lage von Seitenästen, eine Darstellung des Abstands zwischen zwei Bildern von Interesse sowie eine Darstellung der Stentausdehnung,
wobei der Prozessor (106) dafür konfiguriert ist, unter Verwendung von künstlicher Intelligenz zu bestimmen, ob der IVUS-Durchlauf vor einer perkutanen Koronarintervention, PCI, oder nach einer PCI stattfindet.

2. System nach Anspruch 1, wobei der Prozessor (106) ferner dafür konfiguriert ist, ein transversales Querschnittsbild (418) und ein Längsschnittbild (420) des Blutgefäßes auf dem einen oder den mehreren Displays (112) anzuzeigen.

3. System nach Anspruch 2, wobei der Prozessor (106) ferner dafür konfiguriert ist, einen proximalen Referenzpunkt und einen distalen Referenzpunkt zu identifizieren und entlang des Längsschnittbildes (420) einen dem distalen Referenzpunkt (424) entsprechenden Indikator sowie einen dem proximalen Referenzpunkt (426) entsprechenden Indikator anzuzeigen.

4. System nach Anspruch 3, wobei der Prozessor (106) ferner dafür konfiguriert ist, es einem Benutzer zu ermöglichen, die Position der Referenzpunkte zu bestätigen und/oder zu ändern.

5. System nach einem der vorangehenden Ansprüche, wobei der Prozessor (106) dafür konfiguriert ist, die von der Bildgebungsvorrichtung (308) empfangenen Querschnittsbilder zu verarbeiten, um eine Bildsegmentierung für die quantitative Analyse und eine Bildklassifizierung zum automatisierten Identifizieren eines Läsionstyps zu erhalten, und
wobei der Prozessor (106) ferner dafür konfiguriert ist, ein Gefäßprofil auszugeben.

6. System nach einem der vorangehenden Ansprüche, wobei der Prozessor (106) dafür konfiguriert ist, die von der Bildgebungsvorrichtung (308) empfangenen Querschnittsbilder zu verarbeiten, um eine Bildsegmentierung für eine quantitative Analyse und eine Bildklassifizierung zum automatisierten Identifizieren einer Stent-Erfassung zu erhalten, und
wobei der Prozessor (106) ferner dafür konfiguriert ist, ein Gefäßprofil auszugeben.

7. System nach einem der vorangehenden Ansprüche, wobei der Prozessor (106) dafür konfiguriert ist, die von der Bildgebungsvorrichtung (308) empfangenen Querschnittsbilder zu verarbeiten, um eine Bildsegmentierung für eine quantitative Analyse und eine Bildklassifizierung zum automatisierten Identifizieren eines Verkalkungswinkels/-bogens zu erhalten, und
wobei der Prozessor (106) ferner dafür konfiguriert ist, eine Darstellung der Verkalkungslänge auszugeben.

8. System nach einem der vorangehenden Ansprüche, wobei der Prozessor (106) dafür konfiguriert ist, die von der Bildgebungsvorrichtung (308) empfangenen Querschnittsbilder zu verarbeiten, um eine Bildsegmentierung für eine quantitative Analyse und eine Bildklassifizierung zum automatisierten Identifizieren einer Verkalkungsabdeckung zu erhalten, und
wobei der Prozessor (106) ferner dafür konfiguriert ist, eine Darstellung der Verkalkungslänge auszugeben.

9. System nach einem der Ansprüche 1 bis 8, wobei der Prozessor (106) dafür konfiguriert ist, bei einem IVUS-Durchlauf vor einer PCI künstliche Intelligenz zu nutzen, um Indikatoren, die einem proximalen Referenzpunkt, einem distalen Referenzpunkt und der minimalen Lumenfläche entsprechen, automatisch auf der Längsschnittanzeige zu platzieren.

10. System nach einem der Ansprüche 1 bis 9, wobei der Prozessor (106) dafür konfiguriert ist, eine vorgeschlagene Stentlänge und/oder einen vorgeschlagenen Stentdurchmesser für einen Eingriff anzuzeigen.

11. System nach Anspruch 10, wobei der angezeigte vorgeschlagene Stentdurchmesser dem Gefäßdurchmesser an der minimalen Lumenfläche entspricht.

12. System nach einem der vorangehenden Ansprüche, wobei der Prozessor (106) dafür konfiguriert ist, basierend auf der minimalen Lumenfläche ein wünschenswertes, zum Behandeln des Blutgefäßes geeignetes Maß und/oder einen wünschenswerten Prozentsatz einer Stent-Expansion zu bestimmen.

## Revendications

1. Système d'imagerie par échographie intravasculaire, IVUS (100), comprenant:
un cathéter (102) incluant un élément allongé (202) et un embout (204), dans lequel l'élément allongé (202) inclut une gaine (302) avec un axe longitudinal et une lumière (304) avec un noyau d'imagerie (306) disposé dans la lumière (304), dans lequel le noyau d'imagerie (306) inclut un dispositif d'imagerie (308) couplé à une extrémité distale d'une tige d'entraînement (310) pouvant être mise en rotation manuellement ou au moyen d'un mécanisme d'entraînement commandé par ordinateur; et
un module de commande (104) incluant un processeur (106), un générateur d'impulsions (108), une unité d'entraînement (110) et un ou plusieurs dispositifs d'affichage (112);
dans lequel, lors d'une séquence d'IVUS, le noyau d'imagerie (306) est conçu pour se déplacer longitudinalement le long d'un vaisseau sanguin dans lequel le cathéter (102) est inséré pour former une pluralité d'images en coupe transversale le long d'une longueur longitudinale du vaisseau sanguin;
dans lequel le processeur (106) est configuré pour traiter les images en coupe transversale reçues du dispositif d'imagerie (308) à l'aide de réseaux d'apprentissage profond pour obtenir une segmentation d'image pour une analyse quantitative et une classification d'image pour l'identification automatisée de l'un ou d'une combinaison de: type de lésion, détection de stent, identification de la limite de la lumière, identification des dimensions de la lumière, identification de la surface minimale de la lumière, identification de la limite de la média, identification des dimensions de la média, identification de l'angle/arc de calcification, identification de l'étendue de calcification, identification des types de lésions;
dans lequel le processeur (106) est en outre configuré pour fournir en sortie l'un ou une combinaison de: un profil de lumière incluant, par exemple, une coupe longitudinale, un profil de vaisseau incluant, par exemple, une coupe longitudinale, une représentation de la longueur de calcification, l'affichage d'images de référence telles que la surface minimale de la lumière, MLA, la surface minimale du stent, MSA, une représentation de l'emplacement d'une branche latérale, une représentation de la distance entre deux images d'intérêt, une représentation de l'extension du stent;
dans lequel le processeur (106) est configuré pour déterminer si la séquence d'IVUS a lieu avant une intervention coronarienne percutanée, PCI, ou après une PCI, en utilisant l'intelligence artificielle.

2. Système selon la revendication 1, dans lequel le processeur (106) est en outre configuré pour afficher une image en coupe transversale (418) et une image en coupe longitudinale (420) du vaisseau sanguin sur les un ou plusieurs dispositifs d'affichage (112).

3. Système selon la revendication 2, dans lequel le processeur (106) est en outre configuré pour identifier un point de référence proximal et un point de référence distal, et pour afficher un indicateur correspondant au point de référence distal (424) et un indicateur correspondant au point de référence proximal (426) le long de l'image en coupe longitudinale (420).

4. Système selon la revendication 3, dans lequel le processeur (106) est en outre configuré pour permettre à un utilisateur de confirmer et/ou de modifier l'emplacement des points de référence.

5. Système selon l'une quelconque des revendications précédentes, dans lequel le processeur (106) est configuré pour traiter les images en coupe transversale reçues du dispositif d'imagerie (308) pour obtenir une segmentation d'image pour une analyse quantitative et une classification d'image pour l'identification automatisée d'un type de lésion;
et dans lequel le processeur (106) est en outre configuré pour fournir en sortie un profil de vaisseau.

6. Système selon l'une quelconque des revendications précédentes, dans lequel le processeur (106) est configuré pour traiter les images en coupe transversale reçues du dispositif d'imagerie (308) pour obtenir une segmentation d'image pour une analyse quantitative et une classification d'image pour l'identification automatisée de la détection d'un stent;
et dans lequel le processeur (106) est en outre configuré pour fournir en sortie un profil de vaisseau.

7. Système selon l'une quelconque des revendications précédentes, dans lequel le processeur (106) est configuré pour traiter les images en coupe transversale reçues du dispositif d'imagerie (308) pour obtenir une segmentation d'image pour une analyse quantitative et une classification d'image pour l'identification automatisée de l'angle/arc de calcification;
et dans lequel le processeur (106) est en outre configuré pour fournir en sortie une représentation de la longueur de la calcification.

8. Système selon l'une quelconque des revendications précédentes, dans lequel le processeur (106) est configuré pour traiter les images en coupe transversale reçues du dispositif d'imagerie (308) pour obtenir une segmentation d'image pour une analyse quantitative et une classification d'image pour l'identification automatisée de l'étendue de la calcification;
et dans lequel le processeur (106) est en outre configuré pour fournir en sortie: une représentation de la longueur de la calcification.

9. Système selon l'une quelconque des revendications 1 à 8, dans lequel le processeur (106) est configuré, pour une séquence d'IVUS pré-PCI, pour utiliser l'intelligence artificielle pour placer automatiquement des indicateurs correspondant à un point de référence proximal, un point de référence distal et la surface minimale de la lumière sur l'affichage de la coupe longitudinale.

10. Système selon l'une quelconque des revendications 1 à 9, dans lequel le processeur (106) est configuré pour afficher une longueur de stent proposée et/ou un diamètre de stent proposé pour une intervention.

11. Système selon la revendication 10, dans lequel le diamètre de stent proposé affiché correspond au diamètre du vaisseau au niveau de la surface minimale de la lumière.

12. Système selon l'une quelconque des revendications précédentes, dans lequel le processeur (106) est configuré pour déterminer une quantité et/ou un pourcentage souhaitable d'expansion du stent adapté au traitement du vaisseau sanguin sur la base de la surface minimale de la lumière.
